# EUROPEAN PATENT APPLICATION

(11) **EP 4 840 542 A1**
(43) Date of publication of application: **18.11.2026**
(21) Application number: 25771809.8
(22) Date of filing: 12.03.2025
(51) Int. Cl.: B01J 31/28, B01J 37/02, B01J 37/16, C07B 61/00, C07C 67/055, C07C 69/155

(54) **CATALYST FOR PRODUCING ALLYL ACETATE AND METHOD FOR PRODUCING ALLYL ACETATE**

(30) Priority: 15.03.2024 JP 2024041311
(71) Applicant: Crasus Chemical Inc., Oita-shi, Oita 870-0189 (JP)
(72) Inventor: IWAMA, Yasuhiro, Oita-shi, Oita 870-0189 (JP); IGARASHI, Ryo, Oita-shi, Oita 870-0189 (JP); IKUSHIMA, Maiko, Oita-shi, Oita 870-0189 (JP); HOSOGI, Yasuhiro, Oita-shi, Oita 870-0189 (JP)
(74) Representative: Strehl & Partner mbB
(86) International application number: PCT/JP2025/009328
(87) International publication number: WO 2025/192644

(57) **Abstract**

The present invention provides a catalyst which is for producing allyl acetate and which has improved activity during production of allyl acetate from propylene, oxygen, and acetic acid. Provided is a catalyst which is for producing allyl acetate and which is used in production of allyl acetate using propylene, acetic acid, and oxygen as raw materials, wherein (a) palladium, (b) ruthenium, (c) at least one element selected from among copper, nickel, zinc, and cobalt, and (d) an alkali metal salt are supported on (e) a carrier.

## Description

### Technical Field

The present disclosure relates to a catalyst for producing allyl acetate, a method for producing the catalyst, and a method for producing allyl acetate using the catalyst.

### Background Art

Allyl acetate is one of important industrial raw materials used as solvents, raw materials for producing allyl alcohol, and the like. As a method for producing allyl acetate, there is a method of obtaining allyl acetate by a gas phase reaction or a liquid phase reaction using propylene, acetic acid, and oxygen as raw materials. As a catalyst used in this reaction, a catalyst containing palladium as a main catalyst component and an alkali metal compound and/or an alkaline earth metal compound as a co-catalyst component supported on a carrier is known and widely used. For example, JP H2-91045 A (PTL 1) discloses a method for producing allyl acetate using a catalyst in which palladium, potassium acetate, and copper are supported on a carrier.

Further, other catalyst components have been studied, and for example, JP S52-153908 A (PTL 2) discloses a method for producing allyl acetate using a catalyst in which palladium (II) acetylacetonate, molybdenum (VI) dioxyacetylacetonate, and potassium acetate are supported on a carrier, and the activity decrease is suppressed.

JP 2008-279437 A (PTL 3) discloses a method for producing allyl acetate using a catalyst in which palladium, gold, a compound having at least one element selected from copper, nickel, zinc and cobalt, and potassium acetate are supported on a carrier, and the activity decrease is suppressed.

JP H7-029980 B (PTL 4) discloses a method for producing allyl acetate using a catalyst in which palladium, at least one metal selected from copper, lead, ruthenium and rhenium, and potassium acetate are supported on a carrier. However, with respect to the "at least one metal selected from copper, lead, ruthenium and rhenium", only examples of individual metals are described, and there is no example of a case where two or more metals such as copper and ruthenium are combined.

Although the reaction is different from that in the case of allyl acetate, for example, JP 2003-525723 T (PTL 5) discloses a method for producing a catalyst for producing vinyl acetate, in which, in the production of vinyl acetate using ethylene as a starting raw material, a palladium salt is supported on a carrier in a first step, a gold salt is supported on the carrier in a second step, and after a reduction treatment, copper (II) acetate and potassium acetate are supported on the carrier in a third step, thereby suppressing the production of carbon dioxide.

Note that, although catalyst technology for producing vinyl acetate has been established for a long time and attempts have also been made to apply and develop catalyst technology for producing vinyl acetate to catalyst technology for producing allyl acetate, "Catalysts", Vol. 33, No. 1 (1991), pp. 28 to 32 (NPL 1) describes that when the reaction raw material was changed from ethylene to propylene, only results were obtained in which the basic performance of the catalyst was remarkably inferior.

### Citation List

### Patent Literature

PTL 1: JP H2-91045 A
PTL 2: JP S52-153908 A
PTL 3: JP 2008-279437 A
PTL 4: JP H7-029980 B
PTL 5: JP 2003-525723 A

### Non Patent Literature

NPL 1: "Catalysts", Vol. 33, No. 1 (1991), pp. 28 to 32

### Summary of Invention

### Technical Problem

The present disclosure provides a catalyst for producing allyl acetate having improved activity.

### Solution to Problem

The present inventors have found that a catalyst in which at least palladium, ruthenium, at least one element selected from copper, nickel, zinc and cobalt, and an alkali metal salt are supported on a carrier is a catalyst for producing allyl acetate having high activity and improved selectivity.

The present disclosure includes the following aspects [1] to [9].
[1] A catalyst for producing allyl acetate which is used when producing allyl acetate from propylene, acetic acid and oxygen as raw materials, wherein (a) palladium, (b) ruthenium, (c) at least one element selected from copper, nickel, zinc and cobalt, and (d) an alkali metal salt are supported on (e) a carrier.
[2] The catalyst for producing allyl acetate according to aspect [1], wherein an amount of the (b) ruthenium is from 0.8 to 16.7 parts by mass relative to 100 parts by mass of the (a) palladium.
[3] The catalyst for producing allyl acetate according to aspect [1] or [2], wherein a total amount of the (c) at least one element selected from copper, nickel, zinc and cobalt is from 5.0 to 150.0 parts by mass relative to 100 parts by mass of the (a) palladium.
[4] The catalyst for producing allyl acetate according to any one of aspects [1] to [3], wherein the (c) at least one element selected from copper, nickel, zinc and cobalt is at least one selected from copper and zinc.
[5] The catalyst for producing allyl acetate according to any one of aspects [1] to [4], wherein the (d) alkali metal salt is at least one selected from potassium acetate, sodium acetate, and cesium acetate.
[6] The catalyst for producing allyl acetate according to any one of aspects [1] to [5], wherein a mass ratio among the (a) palladium, the (b) ruthenium, a total amount of the (c) at least one element selected from copper, nickel, zinc and cobalt, and the (d) alkali metal salt is (a):(b):(c):(d) = 100:0.8 to 16.7:5.0 to 150.0:157 to 3920.
[7] A method for producing allyl acetate from propylene, acetic acid and oxygen as raw materials, using the catalyst according to any one of aspects [1] to [6].
[8] A method for producing a catalyst for producing allyl acetate, the method comprising the following steps:
   step 1: a step of preparing a solution of a compound containing palladium, a compound containing ruthenium, and a compound containing at least one element selected from copper, nickel, zinc and cobalt, and bringing the solution into contact with (e) a carrier to support the compound containing palladium, the compound containing ruthenium, and the compound containing at least one element selected from copper, nickel, zinc and cobalt on the (e) carrier to obtain a supported carrier (A);
   step 2: a step of contact-impregnating the supported carrier (A) obtained in step 1 with (f) an alkaline solution to obtain an impregnated carrier (B);
   step 3: a step of subjecting the impregnated carrier (B) obtained in step 2 to a reduction treatment to obtain a metal-supported carrier (C); and
   step 4: a step of supporting (d) an alkali metal salt on the metal-supported carrier (C) obtained in step 3.
[9] The method for producing a catalyst for producing allyl acetate according to aspect [8], wherein the compound containing at least one element selected from copper, nickel, zinc and cobalt is copper chloride.

### Advantageous Effects of Invention

According to the present disclosure, a catalyst for producing allyl acetate having improved activity can be provided.

### Description of Embodiments

Preferred embodiments of the present invention will be described below, but it should be understood that the present invention is not limited to these embodiments and that various applications are possible within the spirit and scope of the present invention. In the present disclosure, when "to" is used for a numerical range, the numerical values on both ends are an upper limit and a lower limit, respectively, and are included in the numerical range. When a plurality of upper limits or lower limits are listed, numerical ranges can be formed from all combinations of upper limits and lower limits. Similarly, where a plurality of numerical ranges are listed, upper limits and lower limits can be separately selected and combined from the numerical ranges to form separate numerical ranges.

### Catalyst and Method for Producing Catalyst

A catalyst for producing allyl acetate of an embodiment is a catalyst in which (a) palladium, (b) ruthenium, (c) at least one element selected from copper, nickel, zinc and cobalt, and (d) an alkali metal salt are supported on (e) a carrier. The catalyst for producing allyl acetate may comprise another component supported thereon as necessary. However, preferably, gold is not supported on the catalyst for producing allyl acetate. The use of the catalyst for producing allyl acetate makes it possible to provide allyl acetate in a high space time yield and with high selectivity. As a result, the production cost of allyl acetate can be reduced, and allyl acetate can be efficiently produced.

The catalyst for producing allyl acetate is preferably produced by a method comprising the following steps in this order:
step 1: a step of preparing a solution of a compound containing palladium, a compound containing ruthenium, and a compound containing at least one element selected from copper, nickel, zinc and cobalt, and bringing the solution into contact with (e) a carrier to support the compound containing palladium, the compound containing ruthenium, and the compound containing at least one element selected from copper, nickel, zinc and cobalt on the (e) carrier to obtain a supported carrier (A);
step 2: a step of contact-impregnating the supported carrier (A) obtained in step 1 with (f) an alkaline solution to obtain an impregnated carrier (B);
step 3: a step of subjecting the impregnated carrier (B) obtained in step 2 to a reduction treatment to obtain a metal-supported carrier (C); and
step 4: a step of supporting (d) an alkali metal salt on the metal-supported carrier (C) obtained in step 3.

The method for producing the catalyst for producing allyl acetate is not limited to this method, and the order of step 1 and step 2 may be reversed. However, step 3 must be after step 1 and step 2. Before or after each step, other steps may be included. Hereinafter, these components and steps will be described.

### (a) Palladium

In the present disclosure, the (a) palladium may have any valence, but is preferably metallic palladium. The "metallic palladium" as referred to herein is palladium having a valence of 0. Metallic palladium can be usually obtained by reducing a divalent and/or tetravalent palladium ion with a reducing agent such as hydrazine or hydrogen gas. Substantially all of the (a) palladium is preferably metallic palladium. In the catalyst for producing allyl acetate, not all of the palladium needs to be in a metallic state.

A raw material for the (a) palladium, i.e., a compound containing palladium, is not particularly limited. It is possible to use not only metallic palladium but also a palladium precursor which can be converted into metallic palladium. In the present disclosure, the "compound containing palladium" also encompasses metallic palladium. Examples of the palladium precursor include, but are not limited to, palladium chloride, palladium nitrate, palladium sulfate, sodium chloropalladate, potassium chloropalladate, barium chloropalladate, and palladium acetate. Preferably, sodium chloropalladate is used. The compound containing palladium may be used singly, or two or more types thereof may be used in combination.

Regarding a mass ratio of the (a) palladium to the (e) carrier in the catalyst for producing allyl acetate, the (a) palladium is preferably from 0.1 to 10 parts by mass, more preferably from 0.5 to 5 parts by mass, and still more preferably from 1.0 to 3 parts by mass, relative to 100 parts by mass of the (e) carrier. This ratio is a calculated value calculated from charged amounts of the raw materials. For example, when a palladium precursor is used as the palladium raw material, the ratio is calculated as a ratio of the mass of the palladium element in the palladium precursor to be used to the mass of the carrier.

### (b) Ruthenium

In the present disclosure, the (b) ruthenium may have any valence, but is preferably metallic ruthenium. The "metallic ruthenium" as referred to herein is ruthenium having a valence of 0. Metallic ruthenium can be usually obtained by reducing a trivalent and/or tetravalent ruthenium ion with a reducing agent such as hydrazine or hydrogen gas. Substantially all of the (b) ruthenium is preferably metallic ruthenium. In the catalyst for producing allyl acetate, not all of the ruthenium needs to be in a metallic state.

A raw material for the (b) ruthenium, i.e., a compound containing ruthenium, is not particularly limited. It is possible to use not only metallic ruthenium but also a ruthenium precursor which can be converted into metallic ruthenium. In the present disclosure, the "compound containing ruthenium" also encompasses metallic ruthenium. Examples of the ruthenium precursor include, but are not limited to, ruthenium chloride, carbonyl ruthenium, hexaammineruthenium chloride, dipotassium ruthenium pentachloride, disodium ruthenium pentachloride, dipotassium ruthenium hexachloride, disodium ruthenium hexachloride, and potassium tetraoxoruthenate. Preferably, ruthenium chloride is used. The compound containing ruthenium may be used singly, or two or more types thereof may be used in combination.

Regarding a mass ratio of the (b) ruthenium to the (e) carrier in the catalyst for producing allyl acetate, the (b) ruthenium is preferably from 0.01 to 0.30 parts by mass, more preferably from 0.04 to 0.25 parts by mass, and still more preferably from 0.05 to 0.15 parts by mass, relative to 100 parts by mass of the (e) carrier. This ratio is a calculated value calculated from charged amounts of the raw materials. For example, when a ruthenium precursor is used as the ruthenium raw material, the ratio is calculated as a ratio of the mass of the ruthenium element in the ruthenium precursor to be used to the mass of the carrier.

An amount of the (b) ruthenium in the catalyst for producing allyl acetate is preferably 0.8 parts by mass or more, more preferably 1.7 parts by mass or more, and still more preferably 2.5 parts by mass or more, relative to 100 parts by mass of the (a) palladium. The amount of the (b) ruthenium in the catalyst for producing allyl acetate is preferably 16.7 parts by mass or less, more preferably 15.0 parts by mass or less, and still more preferably 13.0 parts by mass or less, relative to 100 parts by mass of the (a) palladium. The amount of the (b) ruthenium in the catalyst for producing allyl acetate is preferably from 0.8 to 16.7 parts by mass, more preferably from 1.7 to 15.0 parts by mass, and still more preferably from 2.5 to 13.0 parts by mass, relative to 100 parts by mass of the (a) palladium. This ratio is a calculated value calculated from charged amounts of the raw materials. The mass ratio here is a mass ratio of the ruthenium element to the palladium element. When the amount of the ruthenium is within the above ranges, the space time yield of allyl acetate and the allyl acetate selectivity in the allyl acetate production reaction can be obtained in a well-balanced manner.

### (c) At Least One Element Selected from Copper, Nickel, Zinc and Cobalt

In the present disclosure, each element in the (c) at least one element selected from copper, nickel, zinc and cobalt may have any valence, but is preferably an element in a metallic state such as metallic copper, metallic nickel, metallic zinc and metallic cobalt. The "element in a metallic state" as referred to herein is an element having a valence of 0. The element in a metallic state can be obtained by reducing a precursor which can be converted into each metal with a reducing agent such as hydrazine or hydrogen gas. Substantially all of the (c) at least one element selected from copper, nickel, zinc and cobalt is preferably an element in a metallic state. In the catalyst for producing allyl acetate, not all of the copper, nickel, zinc and cobalt needs to be in a metallic state.

A raw material for the (c) at least one element selected from copper, nickel, zinc and cobalt, i.e., a compound containing at least one element selected from copper, nickel, zinc and cobalt, is not particularly limited. It is possible to use not only at least one selected from metallic copper, metallic nickel, metallic zinc and metallic cobalt, but also a precursor which can be converted into a metal of each of the elements. In the present disclosure, the "compound containing at least one element selected from copper, nickel, zinc and cobalt" also encompasses metallic copper, metallic nickel, metallic zinc, and metallic cobalt. As the precursor which can be converted into the metal of each element, soluble salts such as nitrates, carbonates, sulfates, organic acid salts, and halides of these elements can be used. Examples of the organic acid salt include acetate. Examples of the halide include chloride. In general, compounds that are readily available and water soluble are preferred. Preferred examples of the precursor include copper nitrate, copper acetate, nickel nitrate, nickel acetate, zinc nitrate, zinc acetate, cobalt nitrate, cobalt acetate, and copper chloride. Among these, at least one selected from copper acetate and copper chloride is preferred, and copper chloride is most preferred, from the viewpoint of stability of the raw material and availability. The compound containing at least one element selected from copper, nickel, zinc and cobalt may be used singly, or two or more types thereof may be used in combination.

The (c) at least one element selected from copper, nickel, zinc and cobalt is preferably at least one selected from copper and zinc, and more preferably copper.

Regarding a mass ratio of the (c) at least one element selected from copper, nickel, zinc and cobalt to the (e) carrier in the catalyst for producing allyl acetate, a total amount of the (c) at least one element selected from copper, nickel, zinc and cobalt is preferably from 0.05 to 2.0 parts by mass, more preferably from 0.1 to 1.0 parts by mass, and still more preferably from 0.2 to 0.5 parts by mass, relative to 100 parts by mass of the (e) carrier. This ratio is a calculated value calculated from charged amounts of the raw materials. For example, when a chloride is used as the raw material for the at least one element selected from copper, nickel, zinc and cobalt, the ratio is calculated as a ratio of the total mass of copper, nickel, zinc and cobalt elements in the chloride to be used to the mass of the carrier.

A total amount of the (c) at least one element selected from copper, nickel, zinc and cobalt in the catalyst for producing allyl acetate is preferably 5.0 parts by mass or more, more preferably 8.0 parts by mass or more, and still more preferably 16.0 parts by mass or more, relative to 100 parts by mass of the (a) palladium. The total amount of the (c) at least one element selected from copper, nickel, zinc and cobalt in the catalyst for producing allyl acetate is preferably 150.0 parts by mass or less, more preferably 75.0 parts by mass or less, and still more preferably 40.0 parts by mass or less, relative to 100 parts by mass of the (a) palladium. The total amount of the (c) at least one element selected from copper, nickel, zinc and cobalt in the catalyst for producing allyl acetate is preferably from 5.0 to 150.0 parts by mass, more preferably from 8.0 to 75.0 parts by mass, and still more preferably from 16.0 to 40.0 parts by mass, relative to 100 parts by mass of the (a) palladium. The total amount of the (c) at least one element selected from copper, nickel, zinc and cobalt in the catalyst for producing allyl acetate may be from 20.0 to 35.0 parts by mass, or may be from 24.0 to 32.0 parts by mass, relative to 100 parts by mass of the (a) palladium. This ratio is a calculated value calculated from charged amounts of the raw materials. The mass ratio here is a mass ratio of the total amount of at least one element selected from copper, nickel, zinc and cobalt to the palladium element. When the total amount is within the above ranges, the space time yield of allyl acetate and the allyl acetate selectivity in the allyl acetate production reaction can be obtained in a well-balanced manner.

### (d) Alkali Metal Salt

In the present disclosure, the (d) alkali metal salt is at least one compound selected from alkali metal salts. Examples of the alkali metal include lithium, sodium, potassium, rubidium, and cesium. At least one selected from sodium and potassium is preferred, and potassium is more preferred. Examples of the alkali metal salt include hydroxides, acetates, nitrates, and hydrogen carbonates, of alkali metals. Specific examples thereof include sodium hydroxide, potassium hydroxide, lithium acetate, sodium acetate, potassium acetate, rubidium acetate, cesium acetate, lithium nitrate, sodium nitrate, potassium nitrate, rubidium nitrate, cesium nitrate, sodium hydrogen carbonate, and potassium hydrogen carbonate. Among them, at least one selected from potassium acetate, sodium acetate, and cesium acetate is preferred, and at least one selected from potassium acetate and cesium acetate is more preferred.

Regarding a mass ratio of the (d) alkali metal salt to the (e) carrier in the catalyst for producing allyl acetate, the (d) alkali metal salt is preferably from 2.0 to 50 parts by mass, more preferably from 3.0 to 30 parts by mass, and still more preferably from 8.0 to 20 parts by mass, relative to 100 parts by mass of the (e) carrier. This ratio is calculated as a ratio of the mass of the alkali metal salt to be used to the mass of the carrier.

An amount of the (d) alkali metal salt in the catalyst for producing allyl acetate is preferably 157 parts by mass or more, more preferably 200 parts by mass or more, and still more preferably 500 parts by mass or more, relative to 100 parts by mass of the (a) palladium. The amount of the (d) alkali metal salt in the catalyst for producing allyl acetate is preferably 3920 parts by mass or less, more preferably 2000 parts by mass or less, and still more preferably 1000 parts by mass or less, relative to 100 parts by mass of the (a) palladium. The amount of the (d) alkali metal salt in the catalyst for producing allyl acetate is preferably from 157 to 3920 parts by mass, more preferably from 200 to 2000 parts by mass, and still more preferably from 500 to 1000 parts by mass, relative to 100 parts by mass of the (a) palladium. This ratio is a calculated value calculated from charged amounts of the raw materials. The mass ratio here is a mass ratio of the alkali metal salt to the palladium element. When the amount of the alkali metal salt is within the above ranges, the activity of the catalyst in the allyl acetate production reaction and the allyl acetate selectivity can be obtained in a well-balanced manner.

### (e) Carrier

The carrier (e) is not particularly limited. As the (e) carrier, a porous substance generally used as a carrier for a catalyst can be used. Preferred specific examples thereof include silica, alumina, silica-alumina, diatomaceous earth, montmorillonite, titania and zirconia, and silica is more preferred. When a material mainly composed of silica is used as the (e) carrier, a silica content of the (e) carrier is preferably at least 50 mass%, and more preferably at least 90 mass%, relative to the mass of the (e) carrier.

The (e) carrier has a specific surface area preferably in a range of from 10 to 1000 m²/g, and particularly preferably in a range of from 100 to 500 m²/g, as measured by the BET method. A bulk density of the (e) carrier is preferably in a range of from 50 to 1000 g/L, and particularly preferably in a range of from 300 to 500 g/L. A water absorption rate of the (e) carrier is preferably from 0.05 to 3 g/g-carrier, and particularly preferably from 0.1 to 2 g/g-carrier. A pore structure of the (e) carrier preferably has an average pore diameter in a range of from 1 to 1000 nm, and particularly preferably in a range of from 2 to 800 nm. When the average pore diameter is 1 nm or more, gas diffusion can be facilitated. On the other hand, when the average pore diameter is 1000 nm or less, the specific surface area of the carrier necessary for obtaining the catalytic activity can be secured.

Mercury intrusion porosimetry and the gas adsorption method (BJH method) are widely used to measure a pore diameter distribution of the carrier. In terms of classification of pores by IUPAC (International Union of Pure and Applied Chemistry), it is possible to measure macropores of 50 nm or more and some of mesopores of from 2 nm to less than 50 nm by the mercury intrusion porosimetry, and mesopores and micropores of 2 nm or less by the gas adsorption method. An appropriate measurement method can be selected according to the size of the pore diameter.

In the present disclosure, the water absorption rate of the carrier refers to a numerical value as measured by the following measurement method.
1. Precisely weigh about 5 g of the carrier with a balance and place the carrier in a 100-mL beaker. The mass at this time is defined as w1.
2. Add about 15 mL of ion-exchanged water to the beaker to completely cover the carrier.
3. Allow the beaker to stand for 30 minutes.
4. Remove supernatant ion-exchanged water from the carrier.
5. Remove water attached to a surface of the carrier by lightly pressing the carrier with a paper towel until the surface is no longer glossy.
6. Precisely weigh a total mass of the carrier and the ion-exchanged water. The mass at this time is defined as w2.
7. Calculate the water absorption rate of the carrier from the following equation:$Water absorption rate \left(g/g-carrier\right) = \left(w2 - w1\right) / w 1$

Therefore, a water absorption amount (g) of the carrier is calculated by the water absorption rate (g/g-carrier) of the carrier × the mass (g) of the carrier to be used.

A shape of the (e) carrier is not particularly limited. Specific examples of the shape include, but are not limited to, powders, spheres, and pellets. An optimum shape may be selected in accordance with the reaction mode, reactor, etc. to be used.

A particle size of the (e) carrier is also not particularly limited. When the (e) carrier is spherical, a particle diameter thereof is preferably in a range of from 1 to 10 mm, and more preferably in a range of from 2 to 8 mm, in a case where the (e) carrier is used in a fixed-bed tubular reactor for a gas phase reaction. In a case where the catalyst is packed in the tubular reactor and a gas phase reaction is carried out, when the particle diameter is 1 mm or more, an excessive increase in pressure loss during gas flow can be prevented and gas flow can be effectively carried out. On the other hand, when the particle diameter is 10 mm or less, it becomes easy to diffuse a raw material gas to the inside of the catalyst particles, and a catalytic reaction can be effectively carried out.

### (f) Alkaline Solution

The (f) alkaline solution used in step 2 is not particularly limited, and any alkaline solution can be used. The (f) alkaline solution may be a solution of any alkaline compound. Examples of the alkaline compound include hydroxides of alkali metals or alkaline earth metals, bicarbonates of alkali metals or alkaline earth metals, carbonates of alkali metals or alkaline earth metals, and silicates of alkali metals or alkaline earth metals. As the alkali metal, lithium, sodium, and potassium are preferred, and as the alkaline earth metal, barium and strontium are preferred. Among these, preferable examples of the alkaline compound include sodium metasilicate, potassium metasilicate, sodium hydroxide, potassium hydroxide, and barium hydroxide. By contact with the (f) alkaline solution, a part or all of the compound containing palladium, the compound containing ruthenium, and the compound containing at least one element selected from copper, nickel, zinc and cobalt can be converted into an oxide or a hydroxide.

The alkaline compound is appropriately used in excess relative to the total of the (a) palladium, the (b) ruthenium and the (c) at least one element selected from copper, nickel, zinc and cobalt. For example, a product of the molar amount of the alkaline compound and the valence of the alkaline compound is preferably more than 1.1 times and 3.0 times or less, and more preferably more than 1.5 times and 2.0 times or less of the total of a product of the molar amount of the palladium element contained in the compound containing palladium and the valence of palladium, a product of the molar amount of the ruthenium element contained in the compound containing ruthenium and the valence of ruthenium, and a product of the molar amount of the copper element, the nickel element, the zinc element, and the cobalt element contained in the (c) compound containing at least one element selected from copper, nickel, zinc and cobalt and the valence of each of the metals.

A solvent used in forming the solution of the alkaline compound is not particularly limited, and examples thereof include at least one selected from water and alcohols, at least one selected from water, methanol, and ethanol is preferred, and water is particularly preferred.

### Method for Producing Catalyst for Producing Allyl Acetate

Next, each step of the method for producing a catalyst for producing allyl acetate will be described.

### Step 1

In this step, a solution of a compound containing palladium, a compound containing ruthenium, and a compound containing at least one element selected from copper, nickel, zinc and cobalt is prepared, and the solution is brought into contact with (e) a carrier to carry out supporting. The state of these compounds supported on the (e) carrier is preferably a so-called "eggshell-type". In the case of obtaining an eggshell-type supported catalyst, a method of supporting the solution of the compound containing palladium, the compound containing ruthenium, and the compound containing at least one element selected from copper, nickel, zinc and cobalt on the (e) carrier is not particularly limited as long as the method is a method by which an eggshell-type supported catalyst is obtained as a result. The eggshell-type is one of distribution states of active components such as metallic palladium in carrier particles or a formed body, and refers to a state in which most of the active components are present in the vicinity of an outer surface of the carrier particles or the formed body. Specific examples of the method include a method of dissolving the raw material compounds in an appropriate solvent such as water or acetone, or an inorganic acid or an organic acid such as hydrochloric acid, nitric acid, or acetic acid, or a solution thereof, and directly supporting the raw material compounds on a surface layer of the carrier, and a method of indirectly supporting the raw material compounds on the surface layer of the carrier. Examples of the direct supporting method include an impregnation method and a spray method. Examples of the indirect supporting method include, as described later, a method of first supporting a solution of a compound containing palladium, a compound containing ruthenium, and a compound containing at least one element selected from copper, nickel, zinc and cobalt on a carrier (step 1), then moving the compound containing palladium, the compound containing ruthenium, and the compound containing at least one element selected from copper, nickel, zinc and cobalt present inside the carrier particles to the surface layer of the carrier particles by contact impregnation with the (f) alkaline solution (step 2), and then performing reduction (step 3).

The supporting of the compound containing palladium, the compound containing ruthenium, and the compound containing at least one element selected from copper, nickel, zinc and cobalt on the (e) carrier can be carried out by preparing a solution of the compound containing palladium, the compound containing ruthenium, and the compound containing at least one element selected from copper, nickel, zinc and cobalt, and impregnating an appropriate amount of the (e) carrier with the solution. More specifically, the compound containing palladium, the compound containing ruthenium, and the compound containing at least one element selected from copper, nickel, zinc and cobalt are dissolved in an appropriate solvent such as water or acetone, or a solution containing an inorganic acid or an organic acid such as hydrochloric acid, nitric acid, or acetic acid to prepare a solution, and then the carrier is impregnated with the solution to obtain a supported carrier (A). Drying may be performed following impregnation, but it is preferable to proceed to step 2 without a drying step because the drying step can be omitted.

### Step 2

This step is a step of contact-impregnating the supported carrier (A) obtained in step 1 with (f) an alkaline solution to obtain an impregnated carrier (B). Conditions for the contact between the supported carrier (A) and the (f) alkaline solution are not particularly limited, but a contact time is preferably in a range of from 0.5 to 100 hours, and more preferably in a range of from 3 to 50 hours. By setting the contact time to 0.5 hours or more, a desired amount of the catalyst component can be supported, and sufficient catalyst performance can be obtained. When the contact time is 100 hours or less, deterioration of the carrier can be suppressed.

A contact temperature is not particularly limited, but is preferably in a range of from 10 to 80°C, and more preferably in a range of from 20 to 60°C. When the contact temperature is 10°C or higher, a conversion reaction can be sufficiently advanced. When the contact temperature is 80°C or lower, aggregation of palladium, ruthenium, copper, nickel, zinc, and cobalt can be suppressed. The carrier obtained by the contact impregnation with the alkaline solution in this step is referred to as impregnated carrier (B).

### Step 3

This step is a step of subjecting the impregnated carrier (B) obtained in step 2 to a reduction treatment. A reduction reaction is carried out by bringing the impregnated carrier (B) and a reducing agent or a solution thereof into contact with each other. As a reduction method, either liquid phase reduction or gas phase reduction can be used. The metal-supported carrier obtained in this step is referred to as metal-supported carrier (C).

The liquid phase reduction may be carried out either in a non-aqueous system using an alcohol or a hydrocarbon or in an aqueous system. As the reducing agent, for example, a carboxylic acid and a salt thereof, an aldehyde, hydrogen peroxide, a saccharide, a polyhydric phenol, a boron compound, an amine, or hydrazine can be used. Examples of the carboxylic acid and salt thereof include oxalic acid, potassium oxalate, formic acid, potassium formate, potassium citrate, and ammonium citrate. Examples of the aldehyde include formaldehyde and acetaldehyde. Examples of the saccharide include glucose. Examples of the polyhydric phenol include hydroquinone. Examples of the boron compound include diborane and sodium borohydride. Among these, hydrazine, formaldehyde, acetaldehyde, hydroquinone, sodium borohydride, and potassium citrate are preferred, and hydrazine is more preferred.

When the liquid phase method is used to perform reduction, a temperature therefor is not particularly limited, but a liquid phase temperature is preferably in a range of from 0 to 100°C, and more preferably in a range of from 10 to 50°C. When the liquid phase temperature is 0°C or higher, a sufficient reduction rate can be obtained. On the other hand, when the liquid phase temperature is 100°C or lower, aggregation of palladium and ruthenium can be suppressed.

The gas phase reduction is carried out by bringing the impregnated carrier (B) and a reducing gas into contact with each other. Examples of the reducing agent used in the gas phase reduction include at least one selected from the group consisting of hydrogen gas, carbon monoxide, alcohols, aldehydes, and olefins such as ethylene, propylene, and isobutene. The reducing agent is preferably at least one selected from hydrogen gas and propylene.

When the gas phase reduction is performed, a temperature therefor is not particularly limited, but the impregnated carrier (B) is preferably heated to a range of from 30 to 350°C, and more preferably to a range of from 100 to 300°C. When a heating temperature is 30°C or higher, a sufficient reduction rate can be obtained. On the other hand, when the heating temperature is 350°C or lower, aggregation of palladium and ruthenium can be suppressed.

A treatment pressure for a gas phase reduction treatment is not particularly limited, but is preferably in a range of from 0.0 to 3.0 MPaG (gauge pressure), and more preferably in a range of from 0.1 to 1.0 MPaG (gauge pressure), from the viewpoint of equipment.

A feed rate of the reducing agent in the case of performing the gas phase reduction is preferably in a range of from 10 to 15000 hr⁻¹, and particularly preferably in a range of from 100 to 8000 hr⁻¹ in terms of space velocity (hereinafter referred to as SV) in a standard state.

The gas phase reduction may be carried out at any reducing substance concentration, and an inert gas may be added as a diluent as necessary. Examples of the inert gas include helium, argon, and nitrogen gas. The reduction may be carried out using a reducing agent such as hydrogen gas or propylene in the presence of vaporized water.

The catalyst before the reduction treatment may be packed in a reactor, and reduced with propylene gas, and then oxygen and acetic acid may be further introduced to produce allyl acetate. In this case, the supporting of the (d) alkali metal salt in step 4 is carried out between step 2 and step 3.

The metal-supported carrier (C) may be washed with water as necessary. The washing may be performed in a continuous manner, or may be performed batchwise. A washing temperature is preferably in a range of from 5 to 200°C, and more preferably in a range of from 15 to 80°C. A washing time is not particularly limited. It is preferred to select conditions sufficient for removal of residual undesirable impurities. Examples of the undesirable impurities include chlorine-containing compounds. After washing, the metal-supported carrier (C) may be dried by heating, as necessary.

### Step 4

This step is a step of supporting (d) an alkali metal salt on the metal-supported carrier (C) obtained in step 3.

The (d) alkali metal salt can be supported by impregnating the metal-supported carrier (C) with a solution containing a necessary amount of the (d) alkali metal salt and drying it. An amount of the solution used of the (d) alkali metal salt is preferably from 0.9 to 1.0 times by mass the water absorption amount of the carrier. A solvent to be used is not particularly limited. Any solvent can be used as long as it has a solubility sufficient to obtain a solution in an amount of from 0.9 to 1.0 times by mass the water absorption amount of the carrier when the (d) alkali metal salt to be used is dissolved therein. The solvent is preferably water.

A drying temperature and a drying time are not particularly limited.

### Catalyst Component Composition

A mass ratio among (a), (b), (c), and (d) in the catalyst for producing allyl acetate is preferably (a):(b):(c):(d) = 100:0.8 to 16.7:5.0 to 150.0:157 to 3920, and more preferably (a):(b):(c):(d) = 100:1.7 to 15.0:8.0 to 75.0:200 to 2000. The ratios of (a), (b), and (c) are based on the masses of the component elements, and the ratio of (d) is based on the mass of the alkali metal salt.

Supported amounts and a composition ratio of the metal elements contained in the catalyst for producing allyl acetate in the present disclosure can also be measured by chemical analysis such as analysis by a high-frequency inductively coupled plasma atomic emission spectrometer (hereinafter, abbreviated as "ICP"), X-ray fluorescence analysis (hereinafter, abbreviated as "XRF"), or atomic absorption spectrometry.

An example of the measurement method is as follows: a certain amount of the catalyst is ground in a mortar or the like to obtain a uniform powder. Then, the catalyst is added to an acid such as hydrofluoric acid or aqua regia, and the mixture is heated and stirred to dissolve the catalyst, thereby obtaining a uniform solution. Next, the solution is diluted to an appropriate concentration with pure water, and the solution is subjected to quantitative analysis by ICP.

### Method for Producing Allyl Acetate

Hereinafter, a method for producing allyl acetate using the catalyst for producing allyl acetate will be described. The reaction for producing allyl acetate is preferably carried out in a gas phase using propylene, oxygen and acetic acid as raw materials. The type of the gas phase reaction is not particularly limited, and may be a known type. Examples of the type of the gas phase reaction include a fixed bed and a fluidized bed. Preferably, it is practically advantageous to employ a fixed bed flow reaction in which the catalyst for producing allyl acetate is packed in a reaction tube.

A reaction formula is as follows:

CH₂ = CHCH₃ + CH₃COOH + 1/2O₂→ CH₂ = CHCH₂OCOCH₃ + H₂O.

The raw material gas fed to the reactor contains propylene, oxygen gas and acetic acid in a gaseous state, and may further contain nitrogen gas, carbon dioxide, a noble gas or the like as a diluent, as necessary. When propylene, acetic acid, and oxygen are defined as reaction raw materials, a ratio of the reaction raw materials to the diluent is preferably reaction raw materials : diluent = 1:0.05 to 9, and more preferably reaction raw materials : diluent = 1:0.1 to 3 in terms of molar ratio. A ratio among acetic acid, propylene, and oxygen in the raw material gas is preferably acetic acid : propylene : oxygen = 1:1 to 12:0.5 to 2 in terms of molar ratio.

In the reaction for producing allyl acetate, the presence of water in a reaction system is remarkably effective for maintaining the allyl acetate-producing activity and the activity of the catalyst. The raw material gas suitably contains from 0.5 to 23 mol% or from 0.5 to 20 mol% of water vapor.

The propylene used as the reaction raw material is preferably high-purity propylene, but may contain a lower saturated hydrocarbon such as methane, ethane or propane.

Oxygen diluted with an inert gas such as nitrogen gas or carbon dioxide gas (for example, in the form of air) can be fed. However, when the reaction gas is circulated, it is generally advantageous to use a high-concentration oxygen gas, suitably an oxygen gas having a purity of 99 mol% or more.

The propylene and acetic acid used as the reaction raw materials may be produced from fossil raw materials, may be produced from biomass raw materials, or may be a mixture thereof. A concentration of ¹⁴C atoms in propylene, acetic acid, and allyl acetate may be from 1 × 10⁻¹⁴ to 2 × 10⁻¹² relative to the total number of carbon atoms.

A material for the reactor is not particularly limited, but is preferably a material having corrosion resistance.

A reaction temperature is not particularly limited. The reaction temperature is preferably in a range of from 100 to 300°C, and more preferably in a range of from 120 to 250°C. It is practically advantageous from the viewpoint of equipment for the reaction pressure to be in a range of from 0.0 to 3.0 MPaG (gauge pressure), but is not particularly limited. The reaction pressure is more preferably in a range of from 0.1 to 1.5 MPaG (gauge pressure).

When the reaction is carried out by a fixed bed flow reaction, the raw material gas is fed to the catalyst at an SV preferably in a range of from 10 to 15000 hr⁻¹, and more preferably in a range of from 300 to 8000 hr⁻¹ in the standard state.

### Examples

Hereinafter, the present invention will be further described with reference to Examples and Comparative Examples, but is not limited in any way by these descriptions.

### (Example 1) Production of Catalyst A

A commercially available silica spherical carrier (sphere diameter: 5 mm, specific surface area: 155 m²/g, water absorption rate: 0.85 g/g-carrier, bulk specific gravity: 473 g/L, water absorption amount: 402 g/L, hereinafter simply referred to as "silica carrier") was used as a carrier, and a catalyst was produced by the following procedure.

Step 1: 30.3 g of a sodium chloropalladate aqueous solution adjusted to contain 19.79 mass% of palladium, 0.83 g of ruthenium chloride, and 4.6 g of copper chloride dihydrate were mixed, and the volume was made up to 394 mL with pure water. To this solution was added 1 L of the silica carrier, and the carrier was allowed to absorb the entire amount of the solution to obtain a

### supported carrier (A-1).

Step 2: Pure water was added to 63.2 g of sodium metasilicate nonahydrate to dissolve it, and the volume was made up to 804 mL. The supported carrier (A-1) obtained in step 1 was immersed in the solution, and allowed to stand at room temperature for 20 hours to obtain an impregnated carrier (B-1) impregnated with a sodium metasilicate solution.

Step 3: 40.1 g of hydrazine monohydrate was added to a slurry of the impregnated carrier (B-1) obtained in step 2, and the mixture was gently stirred, and then allowed to stand at 25°C for 4 hours. The impregnated carrier (B-1) subjected to the reduction treatment was filtered, then transferred to a glass column equipped with a stopcock, and washed with pure water flowing through the column for 40 hours. Next, drying was performed at 110°C for 4 hours under an air stream to obtain a metal-supported carrier (C-1).

Step 4: Pure water was added to 52 g of potassium acetate to dissolve it, and the volume was made up to 362 mL. The metal-supported carrier (C-1) obtained in step 3 was added to this solution, and the carrier was allowed to absorb the entire amount of the solution. Next, drying was performed at 110°C for 4 hours under an air stream to obtain a catalyst A for producing allyl acetate.

### (Example 2) Production of Catalyst B

A catalyst B was produced by repeating the operation of Example 1, except that the amount of ruthenium chloride was changed from 0.83 g to 0.62 g, that the amount of sodium metasilicate nonahydrate was changed from 63.2 g to 62.2 g, and that the amount of hydrazine monohydrate was changed from 40.1 g to 39.5 g.

### (Example 3) Production of Catalyst C

A catalyst C was produced by repeating the operation of Example 1, except that the amount of ruthenium chloride was changed from 0.83 g to 1.24 g, that the amount of sodium metasilicate nonahydrate was changed from 63.2 g to 65.7 g, and that the amount of hydrazine monohydrate was changed from 40.1 g to 41.5 g.

### (Example 4) Production of Catalyst D

A catalyst D was produced by repeating the operation of Example 1, except that the amount of ruthenium chloride was changed from 0.83 g to 2.06 g, that the amount of sodium metasilicate nonahydrate was changed from 63.2 g to 69.5 g, and that the amount of hydrazine monohydrate was changed from 40.1 g to 44.1 g.

### (Comparative Example 1) Production of Comparative Catalyst E

A comparative catalyst E was produced by repeating the operation of Example 1, except that ruthenium chloride was not added, that the amount of sodium metasilicate nonahydrate was changed from 63.2 g to 59.1 g, and that the amount of hydrazine monohydrate was changed from 40.1 g to 37.5 g.

### (Comparative Example 2) Production of Comparative Catalyst F

Step 1: 30.3 g of a sodium chloropalladate aqueous solution adjusted to contain 19.79 mass% of palladium and 6.13 g of a chloroauric acid aqueous solution adjusted to contain 10 mass% of gold were mixed, and the volume was made up to 382 mL with pure water. To this solution was added 1 L of a silica carrier (bulk specific gravity: 473 g/L, water absorption amount: 402 g/L), and the carrier was allowed to absorb the entire amount of the solution to obtain a supported carrier (A-6).

Step 2: Pure water was added to 35.6 g of sodium metasilicate nonahydrate to dissolve it, and the volume was made up to 803 mL. The supported carrier (A-6) obtained in step 1 was immersed in the solution, and allowed to stand at room temperature for 20 hours to obtain an impregnated carrier (B-6) impregnated with a sodium metasilicate solution.

Step 3: 26.6 g of hydrazine monohydrate was added to a slurry of the impregnated carrier (B-6) obtained in step 2, and the mixture was gently stirred, and then allowed to stand at 25°C for 4 hours. The impregnated carrier (B-6) subjected to the reduction treatment was filtered, then transferred to a glass column equipped with a stopcock, and washed with pure water flowing through the column for 40 hours. Next, drying was performed at 110°C for 4 hours under an air stream to obtain a metal-supported carrier (C-6).

Step 4: Pure water was added to 60 g of potassium acetate and 7.5 g of copper acetate monohydrate to dissolve them, and the volume was made up to 361 mL. The metal-supported carrier (C-6) obtained in step 3 was added to this solution, and the carrier was allowed to absorb the entire amount of the solution. Next, drying was performed at 110°C for 4 hours under an air stream to obtain a comparative catalyst F for producing allyl acetate.

### (Comparative Example 3) Production of Comparative Catalyst G

Step 1: 30.3 g of a sodium chloropalladate aqueous solution adjusted to contain 19.79 mass% of palladium and 1.51 g of a chloroauric acid aqueous solution adjusted to contain 10 mass% of gold were mixed, and the volume was made up to 382 mL with pure water. To this solution was added 1 L of a silica carrier (bulk specific gravity: 473 g/L, water absorption amount: 402 g/L), and the carrier was allowed to absorb the entire amount of the solution to obtain a supported carrier (A-7).

Step 2: Pure water was added to 32.9 g of sodium metasilicate nonahydrate to dissolve it, and the volume was made up to 803 mL. The supported carrier (A-7) obtained in step 1 was immersed in the solution, and allowed to stand at room temperature for 20 hours to obtain an impregnated carrier (B-7) impregnated with a sodium metasilicate solution.

Step 3: 24.9 g of hydrazine monohydrate was added to a slurry of the impregnated carrier (B-7) obtained in step 2, and the mixture was gently stirred, and then allowed to stand at 25°C for 4 hours. The impregnated carrier (B-7) subjected to the reduction treatment was filtered, then transferred to a glass column equipped with a stopcock, and washed with pure water flowing through the column for 40 hours. Next, drying was performed at 110°C for 4 hours under an air stream to obtain a metal-supported carrier (C-7).

Step 4: Pure water was added to 33 g of potassium acetate and 7.5 g of copper acetate monohydrate to dissolve them, and the volume was made up to 361 mL. The metal-supported carrier (C-7) obtained in step 3 was added to this solution, and the carrier was allowed to absorb the entire amount of the solution. Next, drying was performed at 110°C for 4 hours under an air stream to obtain a comparative catalyst G for producing allyl acetate.

### (Comparative Example 4) Production of Comparative Catalyst H

Step 1: Pure water was added to 59.7 g of sodium metasilicate nonahydrate to dissolve it, and the volume was made up to 394 mL to obtain an H-1 solution. To the H-1 solution was added 1 L of a silica carrier (bulk specific gravity: 473 g/L, water absorption amount: 402 g/L), the silica carrier was impregnated with this solution, and allowed to absorb the entire amount of the solution.

Step 2: 30.3 g of a sodium chloropalladate aqueous solution adjusted to contain 19.79 mass% of palladium and 1.24 g of ruthenium chloride were mixed, and the volume was made up to 804 mL with pure water to obtain an H-2 solution. The sodium metasilicate-supported carrier obtained in step 1 was immersed in the H-2 solution and allowed to stand at room temperature for 20 hours to obtain an impregnated carrier.

Step 3: 28.2 g of hydrazine monohydrate was added to a slurry of the impregnated carrier obtained in step 2, and the mixture was gently stirred, and then allowed to stand at 25°C for 4 hours. The obtained impregnated carrier subjected to the reduction treatment was filtered, then transferred to a glass column equipped with a stopcock, and washed with pure water flowing through the column for 40 hours. Next, drying was performed at 110°C for 4 hours under an air stream to obtain a metal-supported carrier.

Step 4: Pure water was added to 52 g of potassium acetate to dissolve it, and the volume was made up to 362 mL. The metal-supported carrier obtained in step 3 was added to this solution, and the carrier was allowed to absorb the entire amount of the solution. Next, drying was performed at 110°C for 4 hours under an air stream to obtain a comparative catalyst H for producing allyl acetate.

### Examples 5 to 8 and Comparative Examples 5 to 8

After 10.5 mL of each of the catalysts A to D and the comparative catalysts E to H obtained in Examples 1 to 4 and Comparative Examples 1 to 4, respectively, was uniformly diluted with 31.5 mL of a silica carrier (bulk specific gravity: 473 g/L, water absorption amount: 402 g/L), the mixture was then packed in a reaction tube (made of SUS316L, internal diameter: 25 mm). A mixed gas having a gas composition of propylene : oxygen : acetic acid : water : nitrogen = 35:6:8.0:23:28.0 (in terms of molar ratio) was introduced at a space velocity of 2070 h⁻¹ under the conditions of a reaction temperature of 150°C and a reaction pressure of 0.75 MPaG (gauge pressure) to perform a reaction of obtaining allyl acetate from propylene, oxygen, and acetic acid. The reaction product was analyzed after 150 hours from the start of the reaction.

As an analysis method in the reaction, a method of cooling the entire amount of an outlet gas having passed through the catalyst-packed bed, recovering the entire amount of a condensed reaction liquid, and analyzing it by gas chromatography was used. As for an uncondensed gas, the entire amount of the uncondensed gas flowing out within the sampling time was measured, and a part thereof was taken out and analyzed by gas chromatography.

The condensed reaction liquid was analyzed using an internal standard method as follows. 0.3 µL of an analytical liquid prepared by adding 1 g of 1,4-dioxane as an internal standard to 6 g of the reaction liquid was injected and analyzed under the following conditions.
Gas chromatography: GC-14B (Shimadzu Corporation)
Detector: FID (H₂ pressure: 60 kPaG, air pressure: 60 kPaG)
Column: capillary column TC-WAX (length: 30 m, internal diameter: 0.25 mm, membrane thickness: 0.25 µm)
Carrier gas: nitrogen gas (flow rate: 30 mL/min)
Temperature conditions: The detector temperature and vaporization chamber temperature were 200°C, the column temperature was held at 40°C for 10 minutes from the start of analysis, then raised to 130°C at a heating rate of 7°C/min, held at 130°C for 20 minutes, then raised to 200°C at a heating rate of 20°C/min, and held at 200°C for 5 minutes.

The uncondensed gas was analyzed using an absolute calibration curve method as follows: 100 mL of an effluent gas was collected and the entire amount thereof was allowed to flow into a 2-mL gas sampler attached to a gas chromatograph under the following conditions.
Gas chromatography: GC-14B available from Shimadzu Corporation
Column: Unibeads IS 60/80 mesh (3 mm ϕ × 3 m)
Carrier gas: helium (flow rate: 20 mL/min)
Temperature conditions: detector temperature: 160°C, vaporization chamber temperature: 150°C, and column temperature: 145°C constant
Detector: TCD (He pressure: 600 kPaG, current: 100 mA)

The activity of the catalyst was calculated as the mass of allyl acetate produced per liter of catalyst volume per hour (space time yield: STY, unit: g/L-cat·hr).

The selectivity of allyl acetate was calculated by the following equation. 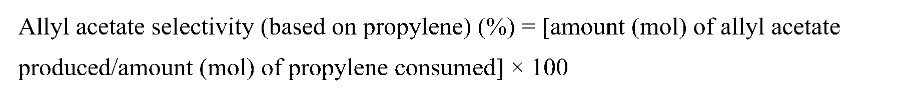 Allyl acetate selectivity (based on propylene) (%) = [amount (mol) of allyl acetate produced/amount (mol) of propylene consumed] × 100

The selectivity of carbon dioxide was calculated by the following equation. 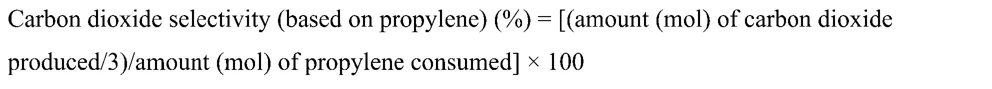 Carbon dioxide selectivity (based on propylene) (%) = [(amount (mol) of carbon dioxide produced/3)/amount (mol) of propylene consumed] × 100

The other by-product selectivity was calculated by the following equation. 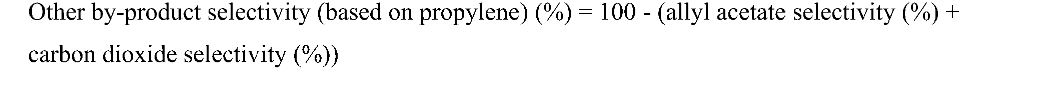 Other by-product selectivity (based on propylene) (%) = 100 - (allyl acetate selectivity (%) + carbon dioxide selectivity (%))

The results of Examples 5 to 8 and Comparative Examples 5 to 8 are shown in Table 1. From Table 1, it can be said that the catalysts of Examples have higher activity than the catalysts of Comparative Examples, and are excellent catalysts.

**Table 1**

| No. | Catalyst | Parts by mass per 100 parts by mass of Pd | | | | | Supported amount per 100 parts by mass of carrier | | | | | Catalyst performance evaluation result | | | |
|---|---|---|---|---|---|---|---|---|---|---|---|---|---|---|---|
| | | Pd | Ru | Cu | Au | Potassium acetate | Pd | Ru | Cu | Au | Potassium acetate | Allyl acetate STY (g/L-cat/h) | Allyl acetate selectivity (%) | CO2 selectivity (%) | Other by-product selectivity (%) |
| Example 5 | Catalyst A | 100 | 6.74 | 28.59 | 0.00 | 867.2 | 1.27 | 0.09 | 0.36 | 0.00 | 10.99 | 563 | 92.6 | 5.2 | 2.2 |
| Example 6 | Catalyst B | 100 | 5.04 | 28.59 | 0.00 | 867.2 | 1.27 | 0.06 | 0.36 | 0.00 | 10.99 | 569 | 92.4 | 5.7 | 1.9 |
| Example 7 | Catalyst C | 100 | 10.08 | 28.59 | 0.00 | 867.2 | 1.27 | 0.13 | 0.36 | 0.00 | 10.99 | 547 | 92.6 | 5.4 | 2.0 |
| Example 8 | Catalyst D | 100 | 16.74 | 28.59 | 0.00 | 867.2 | 1.27 | 0.21 | 0.36 | 0.00 | 10.99 | 539 | 92.8 | 5.3 | 1.9 |
| Comparative Example 5 | Comparative catalyst E | 100 | 0.00 | 28.59 | 0.00 | 867.2 | 1.27 | 0.00 | 0.36 | 0.00 | 10.99 | 531 | 92.6 | 5.2 | 2.2 |
| Comparative Example 6 | Comparative catalyst F | 100 | 0.00 | 39.81 | 10.22 | 1000.6 | 1.27 | 0.00 | 0.50 | 0.13 | 12.68 | 469 | 93.5 | 3.9 | 2.6 |
| Comparative Example 7 | Comparative catalyst G | 100 | 0.00 | 39.81 | 2.52 | 550.3 | 1.27 | 0.00 | 0.50 | 0.03 | 6.98 | 485 | 93.5 | 4.1 | 2.4 |
| Comparative Example 8 | Comparative catalyst H | 100 | 10.08 | 0.00 | 0.00 | 867.2 | 1.27 | 0.13 | 0.00 | 0.00 | 10.99 | 406 | 86.3 | 11.7 | 2.0 |

## Claims

1. A catalyst for producing allyl acetate which is used when producing allyl acetate from propylene, acetic acid and oxygen as raw materials, wherein (a) palladium, (b) ruthenium, (c) at least one element selected from copper, nickel, zinc and cobalt, and (d) an alkali metal salt are supported on (e) a carrier.

2. The catalyst for producing allyl acetate according to claim 1, wherein an amount of the (b) ruthenium is from 0.8 to 16.7 parts by mass relative to 100 parts by mass of the (a) palladium.

3. The catalyst for producing allyl acetate according to claim 1 or 2, wherein a total amount of the (c) at least one element selected from copper, nickel, zinc and cobalt is from 5.0 to 150.0 parts by mass relative to 100 parts by mass of the (a) palladium.

4. The catalyst for producing allyl acetate according to claim 1 or 2, wherein the (c) at least one element selected from copper, nickel, zinc and cobalt is at least one selected from copper and zinc.

5. The catalyst for producing allyl acetate according to claim 1 or 2, wherein the (d) alkali metal salt is at least one selected from potassium acetate, sodium acetate, and cesium acetate.

6. The catalyst for producing allyl acetate according to claim 1 or 2, wherein a mass ratio among the (a) palladium, the (b) ruthenium, a total amount of the (c) at least one element selected from copper, nickel, zinc and cobalt, and the (d) alkali metal salt is (a):(b):(c):(d) = 100:0.8 to 16.7:5.0 to 150.0:157 to 3920.

7. A method for producing allyl acetate from propylene, acetic acid and oxygen as raw materials, using the catalyst according to claim 1 or 2.

8. A method for producing a catalyst for producing allyl acetate, the method comprising the following steps:
step 1: a step of preparing a solution of a compound containing palladium, a compound containing ruthenium, and a compound containing at least one element selected from copper, nickel, zinc and cobalt, and bringing the solution into contact with (e) a carrier to support the compound containing palladium, the compound containing ruthenium, and the compound containing at least one element selected from copper, nickel, zinc and cobalt on the (e) carrier to obtain a supported carrier (A);
step 2: a step of contact-impregnating the supported carrier (A) obtained in step 1 with (f) an alkaline solution to obtain an impregnated carrier (B);
step 3: a step of subjecting the impregnated carrier (B) obtained in step 2 to a reduction treatment to obtain a metal-supported carrier (C); and
step 4: a step of supporting (d) an alkali metal salt on the metal-supported carrier (C) obtained in step 3.

9. The method for producing a catalyst for producing allyl acetate according to claim 8, wherein the compound containing at least one element selected from copper, nickel, zinc and cobalt is copper chloride.
